# EUROPEAN PATENT APPLICATION

(11) **EP 4 438 588 A1**
(43) Date of publication of application: **02.10.2024**
(21) Application number: 22898945.5
(22) Date of filing: 17.11.2022
(51) Int. Cl.: C07C 68/06, C07C 69/96

(54) **METHOD FOR PRODUCING CARBONATE**

(30) Priority: 25.11.2021 KR 20210164916
(71) Applicant: LOTTE CHEMICAL CORPORATION, Seoul 05551 (KR)
(72) Inventor: BAEK, Mi Hwa, Daejeon 34110 (KR); KIM, Wang Gyu, Daejeon 34110 (KR); CHOI, Jong Myung, Daejeon 34110 (KR); HAN, Eun Hye, Daejeon 34110 (KR); KIM, Jin Hyung, Daejeon 34110 (KR)
(74) Representative: Germain Maureau
(86) International application number: PCT/KR2022/018154
(87) International publication number: WO 2023/096263

(57) **Abstract**

The present invention relates to a method for producing a carbonate. The method of the present invention can safely remove a catalyst used in a carbonate production reaction. The method of the present invention can prevent defects that may occur due to the catalyst in the process of purifying the product in the carbonate production reaction. The method of the present invention can produce the carbonate in high yield.

## Description

### [Technical Field]

The present invention relates to a method for producing a carbonate. Particularly, the present invention relates to a method for efficiently removing a catalyst used in a carbonate production reaction.

### [Background Art]

As an organic solvent for a battery electrolyte, ethyl methyl carbonate (EMC) and diethyl carbonate (DEC) are mainly used. The EMC and DEC are prepared by transesterification of dimethyl carbonate (DMC) and ethanol (EtOH). The transesterification is also a reversible reaction. In addition, the transesterification may also be performed in the presence of a catalyst. At this time, a basic catalyst is mainly used as the catalyst. Reaction Formula 1 below shows the transesterification (in which, MeOH is an abbreviation for methanol):

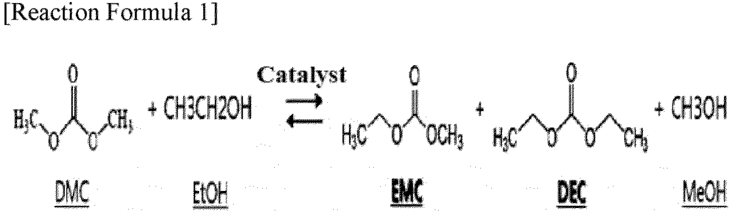

In the reaction, a basic catalyst (mainly a metal salt containing an alkali metal or alkaline earth metal as a metal component) is used as the catalyst. The corresponding catalyst has low solubility in carbonate, which is a reactant or product of the reaction. Therefore, if the catalyst is not separately removed, defects in subsequent processes may occur. This is because the precipitated catalyst causes column plugging or fouling in the subsequent process.

As a method of removing the catalyst, a method of filtering the catalyst through a filter has been considered. However, when the size of the catalyst is smaller than the pore size of the filter, or when alcohol (methanol) contained in the product of the reaction dissolves the catalyst, the catalyst may not be filtered through the filter.

In addition, the catalyst applied to the reaction may include sodium methoxide, sodium ethoxide, or the like, which is a strongly basic and water-reactive material. Accordingly, if the corresponding catalyst is exposed to the atmosphere as it is in the process of removing the catalyst using a filter, etc. or replacing the catalyst, stability problems may also occur.

Patent Document 1 discloses contents of producing dimethyl carbonate through reactive distillation using a NaOCH₃ catalyst and removing the catalyst by applying a metal sintering filter. However, in Patent Document 1, since the catalyst is removed with the filter, safety problems may occur by exposing the catalyst to the atmosphere or moisture in the process of replacing the filter.

### [Prior Arts]

### [Patent Documents]

(Patent Document 1) CN 104557554 A

### [Disclosure]

### [Technical Problem]

An object of the present invention is to safely remove a catalyst used in a carbonate production reaction.

Another object of the present invention is to prevent defects that may occur due to a catalyst in the process of purifying a product in a carbonate production reaction.

Yet another object of the present invention is to produce a carbonate in high yield.

### [Technical Solution]

An aspect of the present invention provides a method for producing a carbonate including: a reaction step of reacting raw materials containing dimethyl carbonate and ethanol in the presence of a catalyst to produce a product containing ethyl methyl carbonate and diethyl carbonate; a catalyst neutralization step of adding a catalyst remover to the product to produce a neutralizing salt of the catalyst and the catalyst remover; and a neutralizing salt removal step of removing the neutralizing salt, in which the catalyst remover has a pKa value of 2.0 or less.

### [Advantageous Effects]

According to the method of the present invention, it is possible to safely remove a catalyst used in a carbonate production reaction.

According to the method of the present invention, it is possible to prevent defects that may occur due to the catalyst in the process of purifying the product in the carbonate production reaction.

According to the method of the present invention, it is possible to produce the carbonate in high yield.

### [Best Mode of the Invention]

Hereinafter, the contents of the present invention will be described in more detail.

As described above, the present invention is intended to efficiently recover or remove a catalyst used in a reaction of producing a carbonate, specifically ethyl methyl carbonate and diethyl carbonate. As a result, the product may be obtained in high purity. In addition, in the present invention, the catalyst may be safely removed. The carbonate produced above may be usefully applied as a solvent for a secondary battery electrolyte.

As described above, in the present invention, the product of the reaction may not dissolve the catalyst applied to the reaction. When the process is performed after the reaction without removing the catalyst, the catalyst is precipitated to block pipes, etc., and cause column plugging or fouling, which is a cause of process defects. The catalyst may also be filtered using a filter, but the catalyst may be dissolved in alcohol, which is a by-product of the reaction, and the dissolved catalyst may pass through the filter as it is. Since a product stream containing the dissolved catalyst also causes post-process defects, it is necessary to remove even the dissolved catalyst. In addition, since the catalyst used in the above-mentioned reaction is a water reactive material, there is a risk of safety accidents when the catalyst is exposed to the atmosphere in the process of replacing the filter, etc.

The present invention is applied with a specific catalyst remover that removes the catalyst applied to the reaction. The catalyst remover may form a salt with the catalyst. As a result, by applying the method of the present invention, it is possible to safely remove the catalyst used in the carbonate production reaction and to produce a carbonate in high purity. In addition, it is possible to secure process economics.

The method for producing the carbonate in the present invention includes at least three steps.

The method for producing the carbonate in the present invention includes a reaction step of reacting raw materials containing dimethyl carbonate and ethanol in the presence of a catalyst to produce a product containing ethyl methyl carbonate and diethyl carbonate.

Although described below, dimethyl carbonate (DMC) and ethanol (EtOH) produces ethyl methyl carbonate (EMC) and diethyl carbonate (DEC) as main products and methanol (MeOH) as a by-product through transesterification.

The conversion rate of the reactants and the selectivity and yield of the target product may vary depending on the composition between the reactants in the raw materials. Therefore, the composition between the reactants in the raw materials may be adjusted in consideration of maximizing a type of target product and the reactant conversion rate.

In an exemplary embodiment, the raw materials may include dimethyl carbonate (DMC) and ethanol (EtOH) at a molar ratio (EtOH/DMC) in the range of 0.2 to 8.0.

Meanwhile, in market prediction data of EMC and DMC, a future demand for EMC is approximately 5 times higher than for DEC. Considering this, it is advantageous to prepare EMC with high selectivity when using the same raw materials. At this time, the raw materials may include DMC and EtOH at a molar ratio (EtOH/DMC) in the range of 0.5 to 2.0.

In addition, since the above-mentioned components are usually present in a liquid form, the raw materials mixed with the components may also contain some moisture. For example, in the raw material producing step, raw materials with the moisture content in the range of 20 ppm to 500 ppm may be reacted.

Since the present invention is intended to remove the catalyst remaining in the product, the reactor for reacting the raw materials is a reactor that leaves at least the catalyst in the reaction product.

Meanwhile, a fixed bed reactor charges and fixes the catalyst in the reactor. In the present invention, the catalyst remaining in the product is removed, but in the fixed bed reactor, no catalyst remains in the product. Accordingly, the reactor mentioned in the present invention is at least not the fixed bed reactor. That is, the reactor mentioned in the present invention is a reactor containing at least a catalyst flowing in the reactor. Such a reactor is also called fluidized bed reactor in the industry.

In the method of the present invention, a continuous flow reactor may be used as the reactor. The continuous flow reactor is literally a reactor in which the supply of reactants and the discharge of products occur continuously.

The continuous flow reactor may be broadly classified into two types. The first type is a continuous stirred tank reactor or mixed flow reactor (CSTR or MFR), and the second type is a plug flow reactor (PFR). Among these, the CSTR may maintain constantly the composition between components in the reactor. In the present invention, a uniform reaction may be performed by applying the CSTR as the reactor. That is, in an exemplary embodiment, the reaction step may be performed by reacting the raw materials in the continuous stirred tank reactor (CSTR).

In the method of the present invention, matters related to the design of the reactor are not particularly limited. In the method of the present invention, the design matters (size, etc.) of the reactor may be determined by comprehensively considering the reaction rate, the yield and/or selectivity of a target product, the conversion rate of the reactants, etc.

The chemical reaction used in the method of the present invention is transesterification of DMC and EtOH. In the chemical reaction, the conversion rate of the reactant, the selectivity and yield of the target product, etc. in the reaction vary depending on the composition of a reactant, the content of a catalyst, a reaction temperature, etc. Here, the catalyst content and the reaction temperature affect the reaction rate.

Meanwhile, in the chemical reaction used in the present invention, as described above, the product composition varies greatly depending on the reactant composition of the raw materials. In addition, when the reaction is performed in a specific reactor, such as a CSTR, the product composition may be particularly determined by the reactant composition. In this case, the catalyst content, the temperature, etc. may not have a significant effect on the composition of the product. When the content and temperature of the catalyst are changed, only the time taken to reach the target product composition is changed, and the final target composition does not change.

In addition, since the conversion rate of the reaction may not be 100%, the product may include a certain amount of DMC and EtOH contained in the raw materials.

In the method of the present invention, the reaction is performed in the presence of the catalyst. The catalyst that may be applied to the reaction may include sodium methoxide (NaOCH₃), sodium ethoxide (NaOC₂H₅), potassium hydroxide (KOH), sodium hydroxide (NaOH), and the like. Among the catalysts, sodium methoxide (or NaOCH₃, Sodium Methoxide; SME) may be used as a representative example. The carbonates in the reactants or products do not dissolve the catalyst, but the alcohols in the reactants or products may dissolve the corresponding catalyst. Accordingly, the product may contain a certain amount of sodium cations (Na⁺). The sodium cations may not be filtered using a general filter, and may be precipitated in a purification process and the like following the reaction to cause process defects. In addition, since the catalyst is a water reactive component, if the catalyst is intended to be removed through a filter without separate treatment as described above, the catalyst may be exposed to the atmosphere in the process of replacing the filter, etc., resulting in a risk of safety accidents.

In an exemplary embodiment, the amount of the catalyst may also be adjusted. In an exemplary embodiment, in the reaction step, a catalyst may be used in the range of 0.02 wt% to 0.2 wt% based on the weight of the dimethyl carbonate. The content of the catalyst may refer to the content of the catalyst itself. For example, the catalyst may be applied as a solution dissolved in a specific solvent, and in this case, the content of the catalyst may refer to the content of the catalyst itself excluding the solvent, not the solution.

The method of the present invention is particularly intended to solve problems occurring depending on the use of the catalyst.

The method for producing the carbonate in the present invention includes a catalyst neutralization step of adding a catalyst remover to the product to produce a neutralizing salt of the catalyst and the catalyst remover. Specifically, in the catalyst neutralization step, the catalyst remover may be subjected to a neutralization reaction with the catalyst to produce a neutralizing salt and methanol, ethanol, or water. Here, the remaining components except for the neutralizing salt vary depending on a type of catalyst. Since the catalyst applied in the reaction is usually a basic component, the catalyst remover that may produce a salt by performing an appropriate neutralization reaction is an acidic component. Specifically, since the catalyst applied in the reaction is a strongly basic component, the catalyst remover that may produce the salt by performing the neutralization reaction is a strongly acidic component. Accordingly, the catalyst remover applied in the method of the present invention has a pKa value of 2.0 or less.

A variety of compounds with the pKa value of 2.0 or less may be applied, but in the present invention, it may be advantageous to use a dicarboxylic acid compound, more specifically a linear dicarboxylic acid compound, as the catalyst remover. Here, the linear dicarboxylic acid may refer to a compound having a structure in which a linear alkylene exists between two carboxyl groups. A representative component of the linear dicarboxylic acid compound is oxalic acid. In the method of the present invention, oxalic acid has been actually used as the catalyst remover.

Since the method of the present invention is applied with a catalyst remover, which is usually an acidic material in the catalyst neutralization step, the pH value of the product to which the catalyst remover is added may also be changed. In an exemplary embodiment, the catalyst neutralization step may be adding the catalyst remover so that the pH of the product to which the catalyst remover is added is in the range of 5 to 8.

In addition, in the method of the present invention, a specific component is applied as the catalyst remover to allow the product to have a target pH even when a relatively small amount of catalyst remover is used. In an exemplary embodiment, the catalyst neutralization step may be adding the catalyst remover in the range of 0.7 to 1 times greater than the weight of the catalyst. Other acidic compounds may be applied as the catalyst remover, but the applied amount is significantly required in order to achieve the desired pH of the product.

The method for producing the carbonate in the present invention includes a neutralizing salt removal step of removing the neutralizing salt produced in the catalyst neutralization step. Here, as a result of removing the neutralizing salt, a reaction product from which the neutralizing salt is removed may be produced. Since the neutralizing salt is present in a solid in the reaction product to cause defects in subsequent processes, the neutralizing salt needs to be removed. That is, before purifying the product containing the neutralizing salt formed by the catalyst in the present invention to obtain the product, the neutralizing salt formed by the catalyst needs to be removed from the product.

In an exemplary embodiment, the method of the present invention removes the neutralizing salt formed by the catalyst in the neutralizing salt removal step. In an exemplary embodiment, the removing of the neutralizing salt may be performed by filtration. When the reaction product properly passes through a filter having an appropriate pore size, the neutralizing salt remains in the filter and the neutralizing salt may be removed from the product.

The method according to the present invention may further include a step of separating the product from which the catalyst has been removed through the contents. Specifically, the method according to the present invention may further include a product separation step of separating ethyl methyl carbonate or diethyl carbonate from the reaction product from which the neutralizing salt has been removed.

Among the products of the chemical reaction used in the method according to the present invention, a target product is carbonate. Currently, DEC is used as a solvent for a secondary battery electrolyte, but as mentioned above, there is also a significant demand for EMC. Therefore, in the method of the present invention, depending on business importance, a process of separating a specific compound (DEC or EMC) from the carbonates including in the product stream as the target product may be performed.

In addition to the steps, the method according to the present invention may include all other steps known to be necessary in the process for preparing the carbonate-based compound and the process used for removing the catalyst used therein.

Hereinafter, the contents of the present invention will be described in more detail with reference to Examples. However, the scope of the present invention is not limited to the following Examples.

### [Experimental Example 1] Karl-Fischer

The moisture content of raw materials was measured using a Karl-Fischer method.

### [Experimental Example 2] Dynamic light scattering analysis

The average size of a neutralizing salt produced by a neutralization reaction of a catalyst remover and a catalyst was measured using dynamic light scattering analysis. The dynamic light scattering analysis was performed using Nano ZS equipment from Malvern. 1 mL of a product sample producing the neutralizing salt was taken, added in a cell of the equipment without pretreatment, and analyzed.

### [Experimental Example 3] Inductively Coupled Plasma Atomic Emission Spectroscopy (ICP-AES)

The Na content was measured using inductively coupled plasma atomic emission spectroscopy (ICP-AES). An Optima 8300 model from Perkin Elmer was used. 10 g of an effluent sample was mixed with 10 ml of 70% nitric acid and 10 ml of water, heated at 250°C for 2 hours, and then analyzed.

### [Experimental Example 4] Gas chromatography

Qualitative and quantitative analysis of a product was performed by gas chromatography (GC). Specifically, GC was performed on a sample in which 1 g of the product was taken and mixed with 0.1 g of m-xylene. An YL6500GC product from YOUNGIN Chromas was used as GC equipment, in which a GC column was DB-1 30 m * 0.32 mm and a GC detector was FID. The conversion rate of dimethyl carbonate, a main reactant of the raw materials, was calculated as mol% of consumption to the added amount. The selectivity of the product, diethyl carbonate was calculated as mol% of the content of diethyl carbonate to the total content of ethyl methyl carbonate and diethyl carbonate. The selectivity of the product, ethyl methyl carbonate was calculated as mol% of the content of ethyl methyl carbonate to the total content of ethyl methyl carbonate and diethyl carbonate.

### [Example 1]

A raw material solution was prepared by mixing 90.1 g (1 mol) of DMC and 46.07 g (1 mol) of EtOH. The moisture content of the raw material was 65 ppm. A catalyst solution was prepared by dissolving a SME catalyst in methanol at a concentration of 30 wt%. The SME catalyst was added to a reactor with a volume of 500 mL along with the raw material solution at a ratio of 0.04 wt% based on the DMC weight of the raw material.

In the reactor, the reaction pressure was maintained at 1 bar, the reaction temperature was maintained at 50°C, and transesterification was performed for 1 hour at a stirring rate of 500 rpm. The conversion rate of DMC in the product was 54 mol%, the selectivity of EMC was 82 mol%, and the selectivity of DEC was 18 mol%.

After completion of the reaction, oxalic acid (OA) was injected into the product as a catalyst remover in an amount of 0.82 times greater than the weight of the catalyst. Thereafter, the pH of the product and the particle diameter of the solid precipitate were analyzed. The particle diameter was measured using dynamic light scattering photometry (DLS). The product was filtered through a syringe filter made of PTFE with a pore size of 1 µm. The sodium content of the filtered product was measured by ICP.

### [Example 2]

The same process as Example 1 was performed, except that a raw material solution with the moisture content of 94 ppm was prepared, and an SME catalyst was added to the reactor at a ratio of 0.05 wt% based on the DMC weight of the raw material.

### [Example 3]

The same process as Example 1 was performed, except that a raw material solution with the moisture content of 130 ppm was prepared, and an SME catalyst was added to the reactor at a ratio of 0.067 wt% based on the DMC weight of the raw material.

### [Example 4]

The same process as Example 1 was performed, except that a raw material solution with the moisture content of 150 ppm was prepared, and an SME catalyst was added to the reactor at a ratio of 0.073 wt% based on the DMC weight of the raw material.

### [Comparative Example 1]

The same process as Example 1 was performed, except that a raw material solution with the moisture content of 91 ppm was prepared, a catalyst solution was added to the reactor at a ratio of 0.05 wt% based on the DMC weight of the raw material, and PPA as a catalyst remover was injected 1.2 times greater than the weight of the catalyst.

### [Comparative Example 2]

The same process as Example 1 was performed, except that a raw material solution with the moisture content of 65 ppm was prepared, a catalyst solution was added to the reactor at a ratio of 0.04 wt% based on the DMC weight of the raw material, and IPA as a catalyst remover was injected 1.6 times greater than the weight of the catalyst.

### [Comparative Example 3]

The same process as Example 1 was performed, except that a raw material solution with the moisture content of 130 ppm was prepared, a catalyst solution was added to the reactor at a ratio of 0.07 wt% based on the DMC weight of the raw material, and IPA as a catalyst remover was injected 1.6 times greater than the weight of the catalyst.

The raw material compositions and experimental results of Examples and Comparative Examples were summarized in Table 1.

**[Table 1]**

| Classifi-cation | | Ex. 1 | Ex. 2 | Ex. 3 | Ex. 4 | Com. Ex. 1 | Com. Ex. 2 | Com. Ex. 3 |
|---|---|---|---|---|---|---|---|---|
| Acid | Type | OA | OA | OA | OA | PPA | IPA | IPA |
| | pKa | 1.25 | 1.25 | 1.25 | 1.25 | 2.16 | 3.46 | 3.46 |
| Moisture content in raw materials (ppm) | | 65 | 94 | 130 | 150 | 91 | 65 | 130 |
| Catalyst content (wt%, based on DMC) | | 0.04 | 0.05 | 0.067 | 0.073 | 0.05 | 0.04 | 0.07 |
| Acid/catalyst (Mass ratio) | | 0.82 | 0.82 | 0.82 | 0.82 | 1.2 | 1.6 | 1.6 |
| pH after acid injection | 5.8 | 5.9 | 6.2 | 6.5 | 7.5 | 8.1 | 8.4 | |
| Na-Salt average particle size (µm) | 1.32 (1.1-2.1) | 1.56 (1.1-2.3) | 1.84 (1.3-2.7) | 1.92 (1.5-2.8) | 2.0 (1.3-3.6) | 2.5 (2.1-3.6) | 2.5 (2.0-3.2) | |
| Na content after filtering (mg/L) | 0.52 | 0.51 | 0.64 | 0.50 | 18.7 | 2.83 | 6.18 | |
| Na-Salt Precipitation in EMC and DEC after distillation at 100°C | X | X | X | X | O | O | O | |
| Na-Salt Solubility in 100 g Water (g, @20°C) | 3.7 | 3.7 | 3.7 | 3.7 | 100 | 37 | 37 | |
| OA: Oxalic Acid | | | | | | | | |
| PPA: Polyphosporic Acid | | | | | | | | |
| IPA: Isophthalic acid | | | | | | | | |
| Na-Salt: Sodium Oxalate, Sodium Polyphosphate, Sodium Isophthalate | | | | | | | | |

According to Table 1, in Examples 1 to 3 applied with OA, which was linear saturated dicarboxylic acid and had a low pKa (acid dissociation constant) of 1.25, it may be seen that the pH of the product was reduced from 12 to 7 even when a small amount of acid was injected. It may also be seen that the particle size of the neutralizing salt produced by adding OA is 1 µm or more.

In addition, it may be seen that the Na content of the product filtered with the filter was 0.50 to 0.64 mg/L, which was similar to the Na content present in the raw material. In other words, it was confirmed that the neutralizing salt produced by the addition of OA was fully removed by the filter. However, it may be seen that in Comparative Examples applied with polyphosphoric acid with a pKa of 2.16 (Comparative Example 1) and isophthalic acid with a pKa of 3.46 (Comparative Examples 2 and 3), the pH of the product was decreased from 12 to about 7 to 8 only by using the catalyst remover 1.5 to 2 times greater than Examples applied with OA.

In addition, the particle size of the neutralizing salt prepared in Comparative Examples was all 1 µm or larger, but the Na content of the filtered product was 2.83 to 6.18 mg/L. In other words, it was confirmed that the neutralizing salt produced by the addition of PPA or IPA was not fully removed by the filter. It is considered because the neutralizing salts produced by PPA and IPA have high solubility in water, so that the product stream dissolves the neutralizing salts.

## Claims

1. A method for producing a carbonate comprising:
a reaction step of reacting raw materials containing dimethyl carbonate and ethanol in the presence of a catalyst to produce a product containing ethyl methyl carbonate and diethyl carbonate;
a catalyst neutralization step of adding a catalyst remover to the product to produce a neutralizing salt of the catalyst and the catalyst remover; and
a neutralizing salt removal step of removing the neutralizing salt,
wherein the catalyst remover has a pKa value of 2.0 or less.

2. The method for producing the carbonate of claim 1, wherein the catalyst remover is linear dicarboxylic acid.

3. The method for producing the carbonate of claim 2, wherein the catalyst remover is oxalic acid.

4. The method for producing the carbonate of claim 1, wherein the reaction step includes reacting raw materials with the moisture content in the range of 20 ppm to 500 ppm.

5. The method for producing the carbonate of claim 1, wherein in the reaction step, the catalyst is used in the range of 0.02 wt% to 0.2 wt% based on the weight of the dimethyl carbonate.

6. The method for producing the carbonate of claim 1, wherein the catalyst neutralization step includes adding the catalyst remover so that the pH of the product to which the catalyst remover is added is in the range of 5 to 8.

7. The method for producing the carbonate of claim 1, wherein the catalyst neutralization step includes adding the catalyst remover in the range of 0.7 to 1 times greater than the weight of the catalyst.

8. The method for producing the carbonate of claim 1, wherein the catalyst is sodium methoxide, sodium ethoxide, potassium hydroxide, or sodium hydroxide.
